# FASCICULE DE BREVET EUROPEEN

(11) **EP 1 156 796 B1**
(45) Date de publication et mention de la délivrance du brevet: **30.11.2005**
(21) Numéro de dépôt: 00907365.1
(22) Date de dépôt: 01.03.2000
(51) Int. Cl.: A61P 7/02, A61K 31/205

(54) **LA GLYCINE BETAINE POUR SON USAGE ANTITHROMBOTIQUE**
DIE GLYCIN-BETAIN FÜR SEINE ANTITHROMBOTISCHE VERWENDUNG
ANTITHROMBOTIC USE OF GLYCINE BETAINE

(30) Priorité: 02.03.1999 BE 9900144
(43) Date de publication de la demande: 28.11.2001
(73) Titulaire: Messadek, Jallal, 4000 Liège (BE)
(72) Inventeur: Messadek, Jallal, 4000 Liège (BE)
(74) Mandataire: Powis de Tenbossche, Roland
(86) Numéro de dépôt international: PCT/BE2000/000021
(87) Numéro de publication internationale: WO 2000/051596

(56) Documents cités:
- EP-A- 0 347 864
- EP-A- 0 781 554
- WO-A-95/15750
- WO-A-97/06795
- WO-A-97/38685
- WO-A-98/19690
- DATABASE CHEMABS [Online] CHEMICAL ABSTRACTS SERVICE, COLUMBUS, OHIO, US ZAPADNYUK, V. I. ET AL: "Bile-secretory effect of trimethylglycine in normal and atherosclerotic animals of different ages" retrieved from STN Database accession no. 107:190742 HCA XP002123170 & BYULL. EKSP. BIOL. MED. (1987), 104(7), 30-2,
- DATABASE CHEMABS [Online] CHEMICAL ABSTRACTS SERVICE, COLUMBUS, OHIO, US PANTELEIMONOVA, T. N. ET AL: "Effect of trimethylglycine on lipid metabolism in rabbits with experimental atherosclerosis" retrieved from STN Database accession no. 99:99080 HCA XP002123171 & FARMAKOL. TOKSIKOL. (MOSCOW) (1983), 46(4), 83-5,
- FAZIO B ET AL: "Treatment of human atherosclerosis with betaine." MINERVA MED, (1961 APR 25) 52 1511-6., XP000853747
- P.H. LIST ET AL.: "HAGERS HANDBUCH DER PHARMAZEUTISCHEN PRAXIS" 1972 , SPRINGER-VERLAG , BERLIN. HEIDELBERG. NEW YORK XP002123167 page 431
- "Betaine for homocystinuria." MEDICAL LETTER ON DRUGS AND THERAPEUTICS, (1997) 39/993 (12)., XP000853853
- WILCKEN D E ET AL: "The natural history of vascular disease in homocystinuria and the effects of treatment." JOURNAL OF INHERITED METABOLIC DISEASE, (1997 JUN) 20 (2) 295-300., XP000853897
- J.E.F. REYNOLDS: "MARTINDALE, The Extra Pharmacopoeia" 1996 , ROYAL PHARMACEUTICAL SOCIETY , LONDON XP002123168 "Betaine hydrochloride" page 1679
- "the merck index" 1996 , MERCK & CO , WHITEHOUSE STATION, NJ XP002123169 "Betaine" page 198
- MCGREGOR D. ET AL NEPHRON vol. 85, 2000, pages 215 - 220
- 'Response to a petition for health Claim from Mr. Emord' FOOD AND DRUG ADMINISTRATION REPORT 28 Novembre 2000,

## Description

L'invention consiste en l'utilisation de la glycine bétaïne pour éliminer les atteintes physiopathologiques vasculaires. L'invention se rapporte à l'activité curative et préventive de la glycine bétaïne dans la pathogénie des maladies thrombo-emboliques et hémostasiques d'origine artérielles ou veineuses.

La glycine bétaïne a une activité préventive en empêchant la formation des thrombi et une activité curative empêchant la prolifération des thrombi en les détruisant. L'intérêt de l'invention réside dans le fait que l'utilisation de la glycine bétaïne ne présente aucun risque hémorragique ou allergique par opposition aux molécules et traitements actuellement utilisés.

### ETAT DE LA TECHNIQUE

Les thromboses vasculaires sont une réponse de l'organisme face à l'agression de la paroi du vaisseau et de son contenu cellulaire et plasmatique. La thrombose est l'activation localisée de la coagulation avec constitution d'un thrombus.

L'intérêt suscité par cette pathologie ces dernières années a permis d'incriminer plusieurs facteurs :
- Le vaisseau , la paroi vasculaire et les cellules endothéliales
- Le rôle des éléments figurés du sang
- Les systèmes de coagulation, de fibrinolyse, et leurs inhibiteurs.

Il existe plusieurs types de thromboses qui peuvent survenir au niveau des artères, des veines, de la microcirculation des organes, des cavités du coeur et des surfaces artificielles en contact avec le sang. Les thromboses vasculaires sont une réponse de l'organisme à l'agression de la paroi du vaisseau et de son contenu cellulaire et plasmatique. La thrombose est une masse organisée d'éléments sanguins (plaquettes, globules rouges et globules blancs), de fibrine et d'autres protéines plasmatiques, qui est déposée à la surface ou qui obstrue la lumière du système vasculaire. Les mécanismes de la thrombose ressemblent à ceux de l'hémostase, mais sont pathologiques par leur localisation intravasculaire anormale. Les thromboses et les embolies sont la cause principale des complications cliniques des maladies cardio-vasculaires et de l'athérosclérose.

D'après Virchow, au moins trois types de facteurs thrombogènes déterminent la localisation, l'extension et la régression d'une thrombose :
- Les facteurs hémodynamiques et rhéologiques;
- La lésion endothéliale;
- L' activation des constituants du sang, en particulier des plaquettes et de la coagulation qui aboutit à la formation de thrombine.
La maladie thrombo-embolique, d'origine artérielle ou veineuse reste une des causes principales de décès dans les pays développés.
La thrombose artérielle est souvent due à une rupture de la plaque d'athérosclérose alors que la thrombose veineuse résulte du déficit d'un inhibiteur de la coagulation (AT III ) ou d'un déficit d'un activateur de la fibrinolyse (protéine S et/ou protéïne C) ou plus fréquemment d'une stase.En effet, si tous les deux résultent d'une interaction entre le sang et la paroi vasculaire, la formation d'une thrombose veineuse et/ou par une anomalie de l'hémostase. La thrombose artérielle est le plus souvent secondaire à une anomalie pariétale et implique principalement les plaquettes sanguines. Elle contribue à une large variété de tableaux cliniques selon les lits artériels intéressés par l'interruption de la vascularisation. La thrombose peut atteindre principalement les artères cardiaques (coronaires), les artères des membres inférieurs, cérébrales ou digestives. Ainsi, la maladie artérielle favorise la formation du thrombus lui même responsable de la majorité des occlusions vasculaires terminales. De plus la participation du désordre de l'hémostase et du thrombus formé à d'autres lésions vasculaires est manifeste : aggravation des lésions de la paroi, ischémie et troubles de la microcirculation.
On peut distinguer trois stratégies thérapeutiques dans la prévention des accidents liés aux thromboses.
Les anticoagulants. Ils constituent l'élément majeur de la prise en charge d'un patient présentant une affection thrombo-embolique. L'héparine et ses dérivés sont couramment utilisés. Cependant, l'utilisation des héparines peut engendrer deux complications majeures, l'hémorragie ou la thrombopénie.

Les anti-vitamines K (AVK). Prescrites pour des traitements au long cours, elles ne peuvent être utilisées dans l'urgence et ne peuvent être prescrites simultanément avec d'autres anti-agrégants dont elles potentialisent l'effet hémorragique.

Les anti-agrégants plaquettaires. Prescrits pour prévenir la thrombose artérielle liée à l'athérosclérose. Actuellement les principaux inhibiteurs du fonctionnement plaquettaire prescrits sont : l'aspirine, la ticlopidine, le dipyridamole, et certains anti-inflammatoires non stéroïdiens comme le flurbiprofène et la prostacycline. Ces traitements possèdent une réelle efficacité toute en présentant des effets indésirables sur les patients à terrains allergiques ou hémorragiques.
Tous ces traitements malgré leur efficacité nécessitent des précautions particulières dans leurs utilisations, telles que l'administration d'antidotes, les problèmes de surdosages et les effets secondaires non désirables. Ces traitements impliquent un suivi nécessaire des patients, dû notamment aux problèmes hémorragiques qui peuvent survenir pendant ou après la médication ainsi qu'aux éventuelles incompatibilités avec d'autres médicaments. Il était donc intéressant de trouver une molécule à haut potentiel antithrombotique sans effets indésirables. De manière tout à fait surprenante la glycine bétaïne est apparue comme possédant un haut potentiel thérapeutique dans le traitement des thromboses.

La glycine bétaïne ou bétaïne de formule (CH3)₃N⁺-CH2-COO- est une molécule connue pour ses propriétés osmoprotectrices ainsi que pour ses utilisations cosmétiques et pharmaceutiques. Diverses applications pharmaceutiques de la bétaïne sont connues et en particulier l'utilisation de la bétaïne pour le traitement de l'homocystinurie cause de troubles cardio-vasculaires (L. & B. Wilken J. Inher. Metab. Dis. 1997). Ainsi les patients souffrant d'homocystinurie, une anomalie génétique, présentent de manière prématurée des troubles athérosclérosiques et thrombo-emboliques (SH. Mudd & al. The metabolic and molecular bases of inherited disease, 1995), ainsi que des maladies cardio-vasculaires (Mc Cully . Atherosclerosis Rev 11, 1983). L'homocystinurie est une déficience héréditaire dont la forme homozygote est rare. On estime que la prévalence de la forme hétérozygote est de 1 pour 200 dans la population générale. L'homocystinurie est due à des taux élevés d'homocystéine dans le plasma des patients atteints. L'administration de la bétaïne permet d'abaisser la concentration d'homocystéine dans le sang.
Dans la publication WO 95 / 157 50, L'auteur afin de prévenir les désordres vasculaires chez les patients homocystéinuriques propose l'usage dans ses ingrédients de la bétaïne.
La publication WO 98 / 19690 s'intéresse également aux patients souffrant d'un taux d'homocystéine élevé dans le sang. L'usage de la bétaïne entre autres ingrédients est destiné à réduire le taux d'homocystéine dans le sang, étant établi que l'homocystéine est un facteur positif de risque dans l'occurence des maladies cardio-vasculaires, ainsi que dans la maladie d'Alzheimer.
La publication EPO 347 864 décrit l'usage de la bétaïne parmi ses ingrédients afin de lutter contre l'augmentation dans le plasma humain des groupes sulfhydryls dus à la cystéine ainsi qu'à l'homocystéine, et ainsi inhiber la formation des plaques d'athérosclérose.
Cette activité anti-athérosclérosique est connue et largement documentée. Ces publications s'intéressent à l'activité de la bétaïne sur le métabolisme des lipides (Zapadnyuk & al. Biol. Med. 1987) ainsi que sur celui du cholestérol (Panteleimonova & al . Farmakol. Toksikol , Moscow 1983).
La publication WO 97 38685 décrit l'utilisation de la bétaïne et de la taurine afin de traiter les complications résultant de l'ischémie dans certains organes. L'ischémie est un arrêt localisé de la circulation sanguine et ne représente qu'une des pathologies dues à la thrombose.
La publication EPO 781 554 décrit dans ses exemples l'expérimentation sur des coeurs énucléés, c'est à dire des coeurs extraits et isolés du système vasculaire. L'utilisation de la bétaïne pour ses propriétés osmoprotectrices et antiradicalaires connues permet ici aux auteurs de revendiquer son rôle protecteur du muscle cardiaque.
D'autres formes de bétaïnes ont été proposées (WO 97 / 06795) sans égaler à ce jour l'activité et les performances de la glycine bétaïne.

Aucune de ces publications ne dévoile l'activité de la glycine bétaïne vis à vis de la thrombose veineuse et/ou artérielle, ni son activité anti-agrégante et anticoagulante.

La glycine bétaïne dans le cadre de la présente invention peut être utilisée pour diverses applications cliniques telles que :
- Thromboses coronariennes et thromboses veineuses
- Infarctus, angine de poitrine, anévrisme, embolie pulmonaire, phlébite
- Les embolies cérébrales
- les chocs post-traumatiques d'origine chirurgicale ou non.
- La prévention des accidents de microcirculation dans les cas suivants : hémophilie, chimiothérapie, âge, contraception orale par les oestrogènes, obésité, tabagisme, prothèse.
- La prévention des risques liés à l'administration des produits de contraste ioniques et non ioniques.

### MATERIEL ET METHODE

### A/ Principe de la thrombose induite par laser. (Seiffge D. et al.., 1989 ; Weichter W. et al., 1983)

Dans ce modèle, la lésion de la paroi vasculaire est induite par un faisceau laser. Ce faisceau entraîne une lésion limitée de l'endothélium vasculaire (seulement 1 à 2 cellules sont détruites). La mise a nu du sous-endothélium, surface thrombogène, amène l'adhésion des plaquettes par l'intermédiaire de la glycoprotéine Ib. Cette adhésion des plaquettes est suivie par leur activation. Elles forment des pseudopodes et sécrètent le contenu de leurs granules. Cette activation entraîne l'apparition de la glycoprotéine IIb-IIIa nécessaire à l'agrégation des plaquettes entre elles. Cette lésion est induite au niveau de la microcirculation mésentérique du rat. Elle est immédiatement suivie par la formation d'un thrombus (quelques secondes). Ce thrombus qui grossit rapidement, sous l'effet du flux sanguin, embolise avant de se former à nouveau.

Dans ce dispositif l'évaluation de l'effet de la glycine bétaïne a été mené conjointement à l'étude de deux molécules pharmacologiquement actives utilisées comme référence: l'acide acétylsalicylique et l'héparine (de bas poids moléculaire). L'évaluation a également porté sur l'activité de la glycine bétaïne par rapport aux effets prothrombotiques induits par les produits de contraste.

### B/ Thrombose induite par stase.

Une laparotomie est effectuée pour dégager la veine cave inférieure sur laquelle on pratique une ligature à To, à To + 2h injection sous cutanée de la glycine bétaïne, à To + 6h prélévement du caillot.

### C/ Protocole expérimental.

Pour ces études on a utilisé des rats Wistar mâles. Leur poids est compris entre 200 et 250 grammes. Après une période de stabulation de 8 jours, les rats sont soumis à un jeûne de 12 heures. Ils sont ensuite anesthésiés, la glycine bétaïne est administrée par voie sous cutanée et le mésentère (laser) ou la veine cave (stase) sont dégagés aux fins des expérimentations.

### EXEMPLES:

### Exemple 1 : Evaluation du nombre d'emboles et de la durée d'embolisation après altération vasculaire par les tirs lasers

| | **Nombre d'emboles** | **Durée d'embolisation (minutes)** |
|---|---|---|
| **Témoin négatif NaCl 0.9%** | 5.33 ±0.58 | 2±0 |
| **Glycine bétaïne 5mg/kg** | 2 ± 0 | 1 ± 0 |
| **Acide acétylsalicylique 100mg/kg** | 1 ± 1 | 0,33 ± 0,58 |
| **Héparine 2 mg/kg** | 2,67 ± 0,58 | 1 ± 0 |

La glycine bétaïne réduit d'une façon significative le nombre d'emboles et le temps d'embolisation après altération vasculaire par des tirs lasers. Ces résultats démontrent sa puissante activité anti-thrombotique.

### Exemple 2 : Evaluation du temps d'hémorragie provoquée ( E. Dejana. Bleeding time in rats. Thrombosis. Rech. 1982)

| | **THP (secondes)** |
|---|---|
| **Témoin négatif NaCl 0.9%** | 101.52 ± 5.7 |
| **Glycine bétaïne 5mg/kg** | 95 ± 5 |
| **Acide acétylsalicylique 100mg/kg** | 276,67 ± 20,82 |
| **Héparine 2 mg/kg** | 313,33 ±20 |

Les résultats montrent que la glycine bétaïne maintient le temps d'hémorragie provoquée dans les valeurs du témoin négatif. La glycine bétaïne en plus de son activité anti-thrombotique n'engendre pas de risques hémorragiques comparativement aux témoins positifs.

### Exemple 3: Evaluation de l'agrégation plaquettaire après altération vasculaire par les tirs lasers (Cardinal & Flower. Pharmacol. Method. 1980)

| | **Amplitude (Ohms)** | **Vélocité (Ohms/min)** |
|---|---|---|
| **Témoin négatif NaCl 0.9%** | 13±1 | 9±1 |
| **Glycine bétaïne 5mg/kg** | 0.66 ±1.15 | 1.66 ± 1.15 |
| **Acide acétylsalicylique 100mg/kg** | 2,33 ± 2.08 | 2 ± 1 |
| **Héparine 2 mg/kg** | 4.33 ± 0.57 | 2.66 ± 0.50 |

Les résultats démontrent l'activité anti-agrégante de la glycine bétaïne.

### Exemple 4 : Evaluation de l'effet de la vis à vis des cellules sanguines

### a/ Dénombrement des plaquettes

| | **Nombre de plaquettes (10**^{**9**}**)** |
|---|---|
| **Témoin négatif NaCl 0.9%** | 788 ± 30.14 |
| **Glycine bétaïne 5mg/kg** | 804.67 ± 20.03 |
| **Acide acétylsalicylique 100mg/kg** | 855.33 ± 63.17 |
| **Héparine 2 mg/kg** | 777.33 ± 6.43 |

### b/ Dénombrement des globules blancs

| | **Nombre des globules blancs (10**^{**9**}**)** |
|---|---|
| **Témoin négatif NaCl 0.9%** | 5 03 ± 0.20 |
| **Glycine bétaïne 5mg/kg** | 4 43± 0.32 |
| **Acide acétylsalicylique 100 mg/kg** | 4.33 ± 1.00 |
| **Héparine 2 mg/kg** | 5.80 ± 0.10 |

### c/ Dénombrement des globules rouges

| | **Nombre des globules rouges (10**^{**12**}**)** |
|---|---|
| **Témoin négatif NaCl 0.9%** | 6.56 ± 0.15 |
| **Glycine bétaïne** 5mg/kg | 6.19 ± 0.25 |
| **Acide acétylsalicylique 100mg/kg** | 6.15 ±0.31 |
| **Héparine 2 mg/kg** | 6.20 ± 0.20 |

Le dénombrement des éléments figurés du sang reste dans les valeurs du témoin négatif et démontre l'innocuité de la glycine bétaïne

### Exemple 5 : Bilan biologique

### a/ Temps de Quick

| | **TQ (secondes)** |
|---|---|
| **Témoin négatif NaCl 0.9%** | 17±1 |
| **Glycine bétaïne 5mg/kg** | 16.9 ± 1.05 |
| **Acide acétylsalicylique 100mg/kg** | 18.33 ± 2.08 |
| **Héparine 2 mg/kg** | 29.50 ± 0.52 |

### b/ Temps de céphaline activée (TCA)

| | TCA (secondes) |
|---|---|
| **Témoin négatif NaCl 0.9%** | 20.5 ± 0.5 |
| **Glycine bétaïne 5mg/kg** | 39.9 ± 1.05 |
| **Acide acétylsalicylique 100mg/kg** | 27.26 ± 1.1 |
| **Héparine 2 mg/kg** | 39 46 ± 1.36 |

### c/ Dosage du fibrinogène

| | **Fibrinogène (g/l)** |
|---|---|
| **Témoin négatif NaCl 0.9%** | 2.45 ± 0.19 |
| **Glycine bétaïne 5mg/kg** | 1.7 ± 0.1 |
| **Acide acétylsalicylique 100mg/kg** | 2.19 ± 0.33 |
| **Héparine 2 mg/kg** | 2.13 ± 0.25 |

### d/ Dosage de l'alpha.2-Antiplasmine (α2AP)

| | **α 2AP (%)** |
|---|---|
| **Témoin négatif NaCl 0.9%** | 30.16 ± 0.85 |
| **Glycine bétaïne 5mg/kg** | 29.7 ± 0.68 |
| **Acide acétylsalicylique 100mg/kg** | 29.36 ± 0.92 |
| **Héparine 2 mg/kg** | 29.4 ± 1.01 |

### e/ Dosage de l'Antithrombine III (AT III)

| | **AT III (%)** |
|---|---|
| **Témoin négatif NaCl 0.9%** | 86 ± 3 |
| **Glycine bétaïne 5mg/kg** | 89.5 ± 1.37 |
| **Acide acétylsalicylique 100mg/kg** | 85.33 ± 3.51 |
| **Héparine 2 mg/kg** | 77.66 ±1.52 |

### Exemple 6: Evaluation de l'activité de la glycine bétaïne en fonction du temps

**Groupes expérimentaux :** Le produit est testé à 5 mg/ kg Thrombose induite par laser

| | |
|---|---|
| Contrôle | NaCl 0,9% |
| Groupe **I** | Le produit est injecté 1 heure avant l'expérimentation |
| Groupe **II** | Le produit est injecté 2 heures avant l'expérimentation |
| Groupe **III** | Le produit est injecté 24 heures avant l'expérimentation |
| Groupe IV | Le produit est injecté 48 heures avant l'expérimentation |

### a) Effet du produit testé ( 5 mg/ml/kg ) sur le Temps d'Hémorragie Provoquée.

| **Groupes** | **T.H.P ( secondes )** |
|---|---|
| **NaCl 0.9 %** | 110 ± 21,2 |
| **I** | 105 ± 26,2 |
| **II** | 145 ± 15,52 |
| **III** | 115,5 ± 14,2 |
| **IV** | 120 ± 10,13 |

### b) Effet du produit testé ( 5 mg/ml/kg ) sur la thrombose artérielle induite par faisceau laser

| **Groupes** | **Nombre de tirs** | **Nombre d'emboles** | **durée d'embolisation (minutes )** |
|---|---|---|---|
| **NACl 0,9 %** | 2,5 ± 0,84 | 5,7 ± 1,5 | 2,1 ± 0,69 |
| **I** | 3,49 ±1,07 | 1,8 ± 1,44 | 0,51 ± 0,5 |
| **II** | 3,0 ±1,5 | 1,4 ± 1,18 | 0,3 ± 0,23 |
| **III** | 2,50 ±1,25 | 1,99 ± 0,4 | 1,00 ± 0,5 |
| **IV** | 2,7 ± 1,0 | 2,2 ± 0,69 | 1,5 ±0,6 |

### c) Effet du produit testé (5 mg/kg) sur l'agrégation plaquettaire induite ex vivo.

| **Groupes** | **Amplitude (Ohms)** | **Vélocité (Ohms / minutes)** |
|---|---|---|
| **NaCl 0,9 %** | 24,23 ± 0,5 | 14,4 ± 2,3 |
| **I** | 11,33 ±3,08 | 8,2 ± 0,2 |
| **II** | 13,2 ±3,5 | 9,3 ± 1,9 |
| **III** | 12,7 ±4,1 | 8,7 ± 1,3 |
| **IV** | 13 ±2,8 | 8,7 ± 1,15 |

### d) Evaluation de l'effet de la glycine bétaïne sur les facteurs de coagulation après administration répétitive sur 5 jours de traitement.

| | **TCA (secondes)** | **Temps de Quick (secondes)** | **Fibrinogène g/l** |
|---|---|---|---|
| **Témoin non traité** | 21,25 ± 2,3 | 16,1 ± 1,0 | 3,03 ± 0,45 |
| **Glycine bétaïne (5mg/kg/j)** | 39,3 ± 2,3 | 19,8 ± 1,2 | 2,2 ± 0,1 |

### Exemple 7 : Evaluation de l'effet de la glycine bétaïne sur la thrombose veineuse induite par stase.

### a) Effet de la glycine bétaïne sur le poids du caillot

| | **Poids du caillot (mg)** |
|---|---|
| **Témoin non traité** | 4,033 ± 2 |
| **Glycine bétaïne (1mg/kg)** | 3,1 ± 0,4 |
| **Glycine bétaïne (2,5 mg/kg)** | 1,63 ± 0,76 |
| **Glycine bétaïne (5mg/kg)** | 0,76 ± 0,4 |

### b) Evaluation de l'effet de la glycine bétaïne sur le plasminogène

| | **Plasminogène %** |
|---|---|
| **NaCl 0,9 %** | 2,7 ± 0,33 |
| **Glycine bétaïne (5mg/kg)** | 1,66 ±0,58 |
| **Glycine bétaïne (2,5mg/kg)** | 2 ± 0,15 |
| **Glycine bétaïne (1mg/kg)** | 2,44 ± 0,58 |

### c) Evaluation de l'effet de la glycine bétaïne sur la coagulation

| | **TCA (secondes)** | **Temps de Quick (secondes)** | **Fibrinogène g/l** |
|---|---|---|---|
| **Témoin non traité** | 30,2 ± 2,7 | 16,1 ± 1,0 | 3,03 ± 0,45 |
| **Glycine bétaïne (1 m g/kg)** | 29,1 ± 2,3 | 16,2 ± 1,2 | 2,63 ± 0,3 |
| **Glycine bétaïne (2,5 mg/kg)** | 31,2 ±2,6 | 16,6 ±0,7 | 2,2 ±0,17 |
| **Glycine bétaïne (5 mg/kg)** | 33,5 ± 1,9 | 15,6 ± 0,4 | 2,32 ± 0,33 |

### d) Evaluation de l'effet de la glycine bétaïne sur les facteurs de coagulation

| | **Anti Xa unité / ml** | **Anti IIa unité / ml** |
|---|---|---|
| **Glycine bétaïne (5 mg/ kg)** | 0,35 ± 0,15 | - |
| **Glycine bétaïne (2,5mg/kg)** | 0,14 ± 0,10 | - |
| **Glycine bétaïne (1 mg / kg)** | 0,08 ± 0,1 | - |

Le traitement avec la glycine bétaïne inhibe les complications thrombo-emboliques déclenchées par les tirs lasers. En effet, ce traitement avec la glycine bétaïne, avant les tirs lasers diminue l'adhésion des plaquettes et leur agrégation au niveau vasculaire.
Le traitement avec la glycine bétaïne inhibe les complications thrombo-emboliques. En effet, ce traitement avec la glycine bétaïne, avant l'induction de la thrombose, a montré un haut potentiel antithrombotique au niveau de tous les paramètres entrant en jeu dans le processus de la formation du thrombus. De plus, les résultats des paramètres biologiques démontrent la parfaite innocuité de la glycine bétaïne qui contrairement aux produits de référence utilisés (aspirine et héparine), n'induit aucun effet hémorragique ni d'effet secondaire indésirable. Ces caractéristiques confèrent à la glycine bétaïne, en plus de son efficacité démontrée, la particularité de pouvoir être administrée aux personnes à risque hémorragique ainsi qu'aux personnes qui présenteraient des risques de sensibilité ou d'allergie face aux traitements antithrombotiques conventionnels (hémophiles, allergiques). La glycine bétaïne ne provoquant de thrombopénies ou désordres hémorragiques (exemples 2 & 4). Le résultat expérimental de l'exemple 5, c, démontre une consommation du fibrinogène.

Il est à noter que, dans les mêmes conditions expérimentales, pour la conservation du sang la glycine bétaïne est apparue comme possédant un haut pouvoir anti-coagulant comparativement à des tubes héparinés ou contenant de l'E.D.T.A. Les doses actives de glycine bétaïne sont apparues entre 3 et 5 mg par tube à hémolyse.Ce résultat expérimental démontre un haut potentiel anticoagulant de la glycine bétaïne. L'utilisation de la glycine bétaïne comme anticoagulant peut être revendiquée, tant pour le traitement du corps humain in vivo, que pour la conservation du sang ex vivo.

### Evaluation de l'activité de la glycine bétaïne vis à vis des produits de contraste.

Dans le cadre de la recherche sur les effets anti-thrombotiques et dans le souci de compléter l'approche de l'efficacité de la glycine bétaïne, nous avons évalué l'effet de la glycine bétaïne sur l'augmentation des risques thrombo-emboliques liés à l'utilisation des produits de contraste connus pour leurs pouvoirs prothrombotiques. L'intérêt représenté par l'utilisation de ce modèle est qu'il permet l'observation directe de la formation du thrombus au site de la lésion vasculaire. Ces résultats expliquent l'apparition d'occlusions thrombotiques lors des angioplasties, surtout chez des patients, dont l'endothélium est déjà endommagé ou lésé. L'angioplastie coronaire cause une dénudation de l'endothélium, exposant le collagène, l'élastine et les cellules musculaires lisses au sang circulant, en analogie avec le modèle de thrombose expérimentale utilisé. Ainsi, l'apparition de nouveaux thrombi est plus élevée chez des patients présentant un infarctus du myocarde récent ou une plaque coronaire excentrique.
L'administration des produits de contraste, diminue le nombre de globules blancs, le nombre de globules rouges et le nombre de plaquettes. Les produits de contraste interagissent avec les leucocytes, induisent la libération de leukotriènes, augmentent la perméabilité vasculaire et exercent un effet chimiotactique. De plus, les produits de contraste agissent sur le contrôle de l'expression de la P-selectine et provoquent l'adhésion des globules blancs à l'endothélium vasculaire. Il a été démontré que l'utilisation des produits de contraste était associée avec l'apparition de thrombi d'importance variable en fonction du produit utilisé.
Deux produits de contrastes ont été étudiés : Hexabrix® (ionique) et Iopamidol ® (non ionique)

### Exemple 11 : Evaluation de l'effet de la glycine bétaïne vis à vis des cellules sanguines

### a/ Dénombrement des plaquettes

| | **Nombre plaquettes (10**^{**9**}**)** |
|---|---|
| **Témoin négatif NaCl 0.9%** | 788.33 ± 30.14 |
| **Hexabrix®** | 620 ± 10 |
| **Iopamidol®** | 585.67 ± 23.54 |
| **Glycine bétaïne 5mg/kg + Hexabrix**® | 669.67 ± 7.37 |
| **Glycine bétaïne 5mg/kg + Iopamidol**® | 704.33 ± 92.33 |

### b/ Dénombrement des globules blancs

| | **Nombre globules blancs (10**^{**12**}**)** |
|---|---|
| **Témoin négatif NaCl 0.9%** | 5.03 ± 0.20 |
| **Hexabrix®** | 2.96 ±0.21 |
| **Iopamidol®** | 3.06 ±0.35 |
| **Glycine bétaïne 5mg/kg + Hexabrix**® | 4.20 ± 0.1 |
| **Glycine bétaïne 5mg/kg + Iopamidol**® | 3.9 ± 0.3 |

### c/ Dénombrement des globules rouges

| | **Nombre globules rouges (10**^{**9**}**)** |
|---|---|
| **Témoin négatif NaCl 0.9%** | 6.56 ± 0.15 |
| **Hexabrix®** | 5.43 ± 0.47 |
| **Iopamidol®** | 5.5 ± 0.36 |
| **Glycine bétaïne 5mg/kg + Hexabrix®** | 6.5 ± 0.15 |
| **Glycine bétaïne 5mg/kg + Iopamidol**® | 6.6 ± 0.19 |

### Exemple 12 : Bilan biologique

### a/ Temps de Quick

| | **TQ (secondes)** |
|---|---|
| **Témoin négatif NaCl 0.9%** | 17 ±1 |
| **Hexabrix®** | 24.13±1 |
| **Iopamidol®** | 28.1 ±0.75 |
| **Glycine bétaïne 5mg/kg + Hexabrix®** | 16.36± 0.56 |
| **Glycine bétaïne 5mg/kg + Iopamidol**® | 17.83 ± 1.2 |

### b/ Temps de céphaline activé (TCA)

| | **TCA (secondes)** |
|---|---|
| **Témoin négatif NaCl 0.9%** | 20.5 ± 0.5 |
| **Hexabrix®** | 49.3 ± 1.85 |
| **Iopamidol®** | 41.33 ±0.8 |
| **Glycine bétaïne 5mg/kg + Hexabrix®** | 25.4 ± 0.61 |
| **Glycine bétaïne 5mg/kg + Iopamidol®** | 22.4 ± 0.7 |

### c/ Dosage du fibrinogène

| | **Fibrinogène (g/l)** |
|---|---|
| **Témoin négatif NaCl 0.9%** | 2.45± 0.19 |
| **Hexabrix®** | 1.49± 0.18 |
| **Iopamidol®** | 1.5 ±0.8 |
| **Glycine bétaïne 5mg/kg + Hexabrix®** | 1 7 ± 0.09 |
| **Glycine bétaïne 5mg/kg + Iopamidol®** | 1.9 ± 0.1 |

### d/ Dosage de l'alpha.2-Antiplasmine (α2AP)

| | α **2AP (%)** |
|---|---|
| **Témoin négatif NaCl 0.9%** | 30.16 ± 0.85 |
| **Hexabrix®** | 23.26 ±1.06 |
| **Iopamidol®** | 25.23 ± 0.95 |
| **Glycine bétaïne 5mg/kg + Hexabrix®** | 25.66 ± 0.64 |
| **Glycine bétaïne 5mg/kg + Iopamidol®** | 28.13 ± 0.8 |

### e/ Dosage de l'Antithrombine III (AT m)

| | **AT III (%)** |
|---|---|
| **Témoin négatif NaCl 0.9%** | 86.3 ± 3 |
| **Hexabrix®** | 81.63 ± 0.66 |
| **Iopamidol®** | 70.6 ±1.51 |
| **Glycine bétaïne 5mg/kg** + **Hexabrix®** | 79.1 ± 1.05 |
| **Glycine bétaïne 5mg/kg** + **Iopamidol®** | 87.26 ± 0.9 |

Le traitement avec la glycine bétaïne inhibe les complications thrombo-emboliques associées à l'utilisation des produits de contraste. En effet, ce traitement avec la glycine bétaïne, avant ou pendant l'injection des produits de contraste, diminue l'adhésion des plaquettes et leur agrégation au niveau vasculaire .Ces résultats démontrent l'effet antithrombotique et thrombolytique de la glycine bétaïne. Il est à noter que les produits de contraste peuvent avoir d'autres effets secondaires tels que la stase sanguine au niveau des cathéters et les lésions endothéliales causées par les procédures d'administrations elles-mêmes. La glycine bétaïne remédie à ces effets indésirables.

### CONCLUSION

La glycine bétaïne possède les mêmes, voire de meilleures, caractéristiques thérapeutiques que les anticoagulants et les anti-agrégants étudiés (acide acétylsalicylique et l'héparine), tout en ne présentant aucun effet indésirable.

Les performances supérieures en terme d'efficacité thérapeutique de la glycine bétaïne par rapport à ces deux molécules (acide acétylsalicylique et héparine) incitent à la formulation d'un médicament ayant pour principe thérapeutiquement actif la glycine bétaïne. Ce médicament étant destiné au traitement des thromboses et des affections thrombo-emboliques.

Selon les résultats exposés ce médicament revendique aussi les indications anticoagulant, anti-agrégant et fibrinolytique. L'innocuité démontrée de cette molécule permet d'envisager des traitements à long terme sans pour autant nécessiter une surveillance biologique.

L'intérêt de l'utilisation de la glycine bétaïne réside dans le fait qu'elle agit à plusieurs niveaux de l'hémostase à savoir au niveau de l'agrégation plaquettaire, au niveau de la coagulation et au niveau fibrinolytique. Cette activité est durable et évite des administrations répétitives ce qui constitue une amélioration notable par rapport aux traitements existant. L'administration de la bétaïne n'induit aucun risque hémorragique, ni autres effets secondaires (ex : thrombopénie induite à l'héparine) ce qui constitue une avancée majeure en thérapeutique antithrombotique.

### Exemple 8 : Evaluation du nombre d'emboles et de la durée d'embolisation après altération vasculaire par les tirs lasers et administration des produits de contrastes.

| | **Nombre d'emboles** | **Durée d'embolisation (minutes)** |
|---|---|---|
| **Témoin négatif** NaCl **0.9%** | 5.33 ± 0.58 | 2 ± 0 |
| **Hexabrix®** | 8 ± 1 | 3.67 ±0.58 |
| **Iopamidol®** | 11.67± 0.50 | 6.33 ± 0,52 |
| **Glycine bétaïne 5mg/kg + Hexabrix®** | 4 ± 1 | 2 ± 0 |
| **Glycine bétaïne 5mg/kg + Iopamidol**® | 5.33 ± 0.58 | 2.33 ± 0.48 |

### Exemple 9 : Evaluation du temps d'hémorragie provoquée (THP)

| | **THP (secondes)** |
|---|---|
| **Témoin négatif NACl 0.9%** | 101.52 ±5.7 |
| **Hexabrix®** | 195 ± 13.23 |
| **Iopamidol®** | 128 ± 7.64 |
| **Glycine bétaïne 5mg/kg + Hexabrix®** | 150 ± 5 |
| **Glycine bétaïne 5mg/kg + Iopamidol®** | 111 ± 6.60 |

### Exemple 10: Evaluation de l'agrégation plaquettaire après altération vasculaire par les tirs lasers

| | **Amplitude (Ohms)** | **Vélocité (Ohms/min)** |
|---|---|---|
| **Témoin négatif Nacl 0.9%** | 13 ± 1 | 9 ± 1 |
| **Hexabrix®** | 6±1 | 5.66 ±0.57 |
| **Iopamidot®** | 15 ±2.64 | 12.33 ± 0,50 |
| **Glycine bétaïne 5mg/kg + Hexabrix®** | 2 ± 1 | 5 ± 0 |
| **Glycine bétaïne 5mg/kg + Iopamidol®** | 4.66 ± 0.52 | 9.33 ± 0.8 |

## Revendications

1. Utilisation de la glycine bétaïne pour l'obtention d'un médicament destiné à traiter les thromboses non induites par l'homocystinurie.

2. Utilisation de la glycine bétaïne pour l'obtention d'un médicament destiné à traiter les affections thrombo-emboliques non induites par l'homocystinurie.

3. Utilisation de la glycine bétaïne pour l'obtention d'un médicament destiné à traiter les désordres de la coagulation du sang chez un patient.

4. Utilisation de la glycine bétaïne pour l'obtention d'un médicament destiné à traiter les désordres de l'agrégation plaquettaire chez un patient.

5. Utilisation de la glycine bétaïne pour l'obtention d'un médicament destiné à lyser un thrombus.

6. Utilisation de la glycine bétaïne pour l'obtention d'un médicament destiné à traiter les thromboses chez les sujets à risque hémorragique.

7. Utilisation de la glycine bétaïne comme agent anticoagulant pour la conservation du sang ex-vivo.

8. Utilisation de la glycine bétaïne pour l'obtention d'un médicament destiné à contrecarrer les effets thrombo-emboliques induits par les produits de contraste.

9. Utilisation de la glycine bétaïne selon la revendication 8, **caractérisée en ce qu'**elle puisse être simultanément administrée avec des produits de contraste à un patient.

10. Utilisation de la glycine bétaïne comme principe actif selon l'une quelconque des revendications 1 à 9, **caractérisée en ce qu'**une quantité efficace de glycine bétaïne est associée à un support, véhicule ou excipient pharmaceutiquement acceptable.

11. Utilisation selon l'une quelconque des revendications 1 à 6, 8 et 9, pour la préparation d'un médicament pour administration par voie sous-cutanée.

## Patentansprüche

1. Gebrauch der Glycinbetain für die Erlangung eines Medikaments zur Behandlung von Thrombosen, die nicht von Homocystinuria verursacht werden.

2. Gebrauch der Glycinbetain für die Erlangung eines Medikaments zur Behandlung von thromboembolischen Erkrankungen, die nicht von Homocystinuria verursacht werden.

3. Gebrauch der Glycinbetain für die Erlangung eines Medikaments zur Behandlung von Blutgerinnungsstörungen bei einem Patienten.

4. Gebrauch der Glycinbetain für die Erlangung eines Medikaments zur Behandlung von Blutlättchenaggregationsstörungen bei einem Patienten.

5. Gebrauch der Glycinbetain für die Erlangung eines Medikaments, der dazu bestimmt ist, einen Thrombus zu lysieren.

6. Gebrauch der Glycinbetain für die Erlangung eines Medikaments zur Behandlung von Thrombosen bei Patienten, die ein Risiko für Hämorrhagien zeigen.

7. Gebrauch der Glycinbetain als blutgerinnungshemmendes Agens für die ex vivo Blutbewahrung.

8. Gebrauch der Glycinbetain für die Erlangung eines Medikaments zur Entgegenwirkung von thromboembolischen Effekten, die auf Kontrastmittel zurückzuführen sind.

9. Gebrauch der Glycinbetain nach Anspruch 8, mit dem weiteren Kennzeichen, dass sie gleichzeitig mit Kontrastmitteln dem Patienten verabreicht werden kann.

10. Gebrauch der Glycinbetain als Wirkstoff nach einem der Ansprüche 1 bis 9, mit dem weiteren Kennzeichen, dass eine wirksame Menge von Glycinbetain mit einem pharmazeutisch annehmbaren Träger, Vehikel oder Trägersubstanz verbunden wird.

11. Gebrauch nach einem der Ansprüche 1 bis 6, 8 und 9, für das Präparat eines Medikaments für eine subkutane Verabreichung.

## Claims

1. The use of glycine betaine for obtaining a drug intended for the treatment of thromboses which are not induced by homocystinuna.

2. The use of glycine betaine for obtaining a drug intended for the treatment of thrombo-embolic diseases which are not induced by homocystinuria

3. The use of glycine betaine for obtaining a drug intended for the treatment of blood coagulation disorders in a patient

4. The use of glycine betaine for obtaining a drug intended for the treatment of platelet aggregation disorders in a patient.

5. The use of glycine betaine for obtaining a drug intended for the lysis of a thrombus.

6. The use of glycine betaine for obtaining a drug intended for the treatment of thromboses in subjects at risk of haemorrhage.

7. The use of glycine betaine as an anticoagulant agent for the preservation of ex-vivo blood.

8. The use of glycine betaine for obtaining a drug intended for counteracting thrombo-embolic effects induced by contrasting products.

9. The use of glycine betaine according to claim 8, **characterised in that** it can be administered to a patient simultaneously with contrasting products.

10. The use of glycine betaine as active ingredient according to any one of claims 1 to 9, **characterised in that** a effective amount of glycine betaine is associated with a pharmaceutically acceptable support, vehicle or excipient

11. The use according to any one of the claims 1 to 6, 8 and 9, for the preparation of a drug for subcutaneous administration.
